# EUROPÄISCHE PATENTSCHRIFT

(11) **EP 2 726 471 B1**
(45) Veröffentlichungstag und Bekanntmachung des Hinweises auf die Patenterteilung: **18.05.2016**
(21) Anmeldenummer: 12729611.9
(22) Anmeldetag: 25.06.2012
(51) Int. Cl.: C07D 307/28, C07D 307/93, C07D 311/94

(54) **VERFAHREN ZUR HERSTELLUNG CYCLISCHER ENOLETHER**
PROCESS FOR THE PREPARATION OF CYCLIC ENOLETHERS
PROCEDÉ POUR LA SYNTHÈSE D'ÉNOL ÉTHER CYCLIQUES

(30) Priorität: 30.06.2011 EP 11172082
(43) Veröffentlichungstag der Anmeldung: 07.05.2014
(73) Patentinhaber: BASF SE, 67056 Ludwigshafen am Rhein (DE)
(72) Erfinder: EBEL, Klaus, 68623 Lampertheim (DE); BRUNNER, Bernhard, 69120 Heidelberg (DE); STOCK, Christoph, 67158 Ellerstadt (DE); PELZER, Ralf, 37699 Fürstenberg (DE)
(74) Vertreter: BASF IP Association
(86) Internationale Anmeldenummer: PCT/EP2012/062183
(87) Internationale Veröffentlichungsnummer: WO 2013/000846

(56) Entgegenhaltungen:
- WO-A1-2008/066299
- WO-A1-2008/133441
- JP-A- 2010 095 447
- US-A- 4 056 541
- MICHÈLE DANET ET AL: "Enantioselective Synthesis of the Originally Proposed Usneoidone Structure: Evidence for a Structural Revision", EUROPEAN JOURNAL OF ORGANIC CHEMISTRY, Bd. 2004, Nr. 9, 1. Mai 2004 (2004-05-01), Seiten 1911-1922, XP055003702, ISSN: 1434-193X, DOI: 10.1002/ejoc.200300767
- BONIFAZI E L ET AL: "Antiproliferative activity of synthetic naphthoquinones related to lapachol. First synthesis of 5-hydroxylapachol", BIOORGANIC & MEDICINAL CHEMISTRY, PERGAMON, GB, Bd. 18, Nr. 7, 1. April 2010 (2010-04-01), Seiten 2621-2630, XP027027269, ISSN: 0968-0896 [gefunden am 2010-02-23]
- ALAN F. THOMAS ET AL: "Homologues ofp-Menthane Derivatives in Roman Camomile", HELVETICA CHIMICA ACTA, Bd. 64, Nr. 5, 22. Juli 1981 (1981-07-22), Seiten 1488-1495, XP055003897, ISSN: 0018-019X, DOI: 10.1002/hlca.19810640524
- ZAKHARKIN L. L. ET AL.: "Syntheses of 2-oxabicyclo [ 4.10.0 ] hexadec- 1 (6)-enefrom cyclododeeanone", RUSSIAN CHEMICAL BULLETIN, Bd. 43, Nr. 4, 4. April 1994 (1994-04-04), Seiten 608-611, XP002654577,

## Beschreibung

Die vorliegende Erfindung betrifft ein Verfahren zur Herstellung von cyclischen Enolethern.

Cyclische Enolether stellen bei der Synthese von makrocyclischen Lactonen, die als Riechstoffe verwendet werden, wichtige Zwischenprodukte dar. So wird in
US 3,890,353 die Herstellung des gesättigten 15-Pentadecanolid (Exaltolide^{®}) der Formel (a) beschrieben, 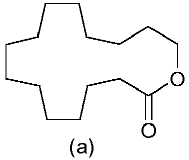 wobei als Ausgangsprodukt der cyclische Enolether 13-Oxa-1.12-didehydro-bicyclo[10.4.0] hexadecan der Formel (b) dient.

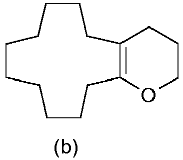

Neben den voran beschriebenen 16-Ring Lactonen sind auch 15-Ring Lactone als moschusähnliche Riechstoffe beschrieben. So werden in EP 0 862 911 gesättigte und ungesättigte 15-Ring Lactone der Formeln (d1) und (d2) beschrieben, wobei R gleich Methyl oder Wasserstoff ist.

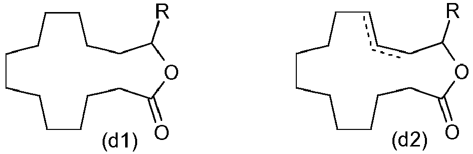

Die 15-Ring Lactone können ausgehend vom entsprechenden cyclischen Enolether der Formel (e) hergestellt werden. 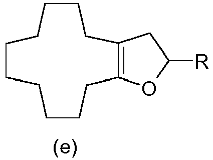 mit R gleich H oder Me Die Herstellung von cyclischen Enolethern, die als Riechstoffe oder zur Herstellung von Riechstoffen geeignet sind, wird beispielsweise in GB 1266092, DE 2136496, US 3,890,353, DE 25 11 410, DE 29 06 296 oder JP 2010-95447 beschrieben.

### ** S.2a

Teils sind die Ausgangsprodukte zur Herstellung der cyclischen Enolether nicht einfach zugänglich beziehungsweise die Herstellung der cyclischen Enolether verläuft über mehrstufige Synthesen mit zum Teil schlechten Ausbeuten, teils sind die Reaktionsbedingungen für die Cyclisierung recht drastisch, beispielsweise durch Verwendung größerer Mengen konzentrierter Schwefelsäure, oder die Aufarbeitung der cyclischen Enolether ist in wirtschaftlicher Sicht noch nicht zufrieden stellend oder es werden große Überschüsse von Reagenzien oder Ausgangsstoffen benötigt.

Ausgehend von diesem Stand der Technik bestand die Aufgabe darin, flexible und effizientere Synthesewege zu solchen Riechstoffen zu finden.

Diese Aufgabe wird durch ein Verfahren zur Herstellung von cyclischen Enolethern der Formeln (I) und/oder (II) 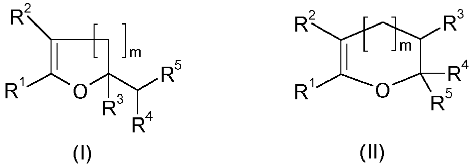 durch Cyclisierung einer Ausgangsverbindung der Formel (III) 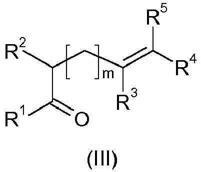 worin
- m: gleich null (0), eins (1) oder zwei (2) ist,
- R¹: ein C₁-C₁₀-Alkylrest ist,
- R²: ein C₁-C₁₀-Alkylrest_ist,
oder die Reste R¹ und R² zusammen für eine zweibindige Gruppe -(CH₂)ₓ- steht, wobei x eine ganze Zahl von 3 bis 12 bedeutet,
- R³: Wasserstoff oder Methyl ist,
- R⁴: Wasserstoff oder Methyl ist, und
- R⁵: Wasserstoff oder Methyl ist,

in Gegenwart einer Brönstedt- oder Lewis-Säure, wobei die Reaktion als Reaktivdestillation durchgeführt wird, wobei die gebildeten cyclischen Enolether der Formeln (I) und/oder (II) destillativ aus dem Reaktionsgemisch von der Ausgangsverbindung der Formel (I II) abgetrennt werden, gelöst.

Bei den im erfindungsgemäßen Verfahren verwendbaren Brönstedt-Säuren handelt es sich sowohl um organische als auch um anorganische Säuren. Bevorzugt werden Brönstedt-Säuren, die selbst nicht mit der Ausgangsverbindung der Formel (III) reagieren können, das heißt in einer Reaktion verbraucht werden, sondern lediglich als Protonenquelle für eine durch Protonen katalysierte chemische Reaktion dienen. Nicht einschränkende Beispiele für besonders geeignete Brönstedt-Säuren sind Schwefelsäure, Phosphorsäure, Methansulfonsäure, p-Toluolsulfonsäure, starke saure Ionenaustauscher, Tetrafluoroborsäure, Trifluoressigsäure, Ameisensäure oder Oxalsäure.

Als Lewis-Säure können in dem erfindungsgemäßen Verfahren beispielsweise Aluminiumtrichlorid, Zinntetrachlorid, Titantetrachlorid, Zirkoniumtetrachlorid, Eisentrichlorid oder Nickeldichlorid eingesetzt werden.

Die Menge der Brönstedt- oder Lewis-Säure, die im erfindungsgemäßen Verfahren verwendet wird, kann in einem breiten Bereich variiert werden. Prinzipiell kann das molare Verhältnis der Brönstedt- oder Lewis-Säure zur Verbindung der Formel (III) größer, gleich oder kleiner als 1 sein. Prinzipiell reichen Spuren von Säure, um die Cyclisierung zu katalysieren.

Bevorzugt ist in dem erfindungsgemäßen Verfahren das molare Verhältnis der Brönstedt- oder Lewis-Säure, insbesondere der Brönstedt-Säure zur Verbindung der Formel (III) nicht größer als 1, besonders bevorzugt nicht größer als 0,15, ganz besonders bevorzugt zwischen 0,1 und 0,0005, insbesondere zwischen 0,07 und 0,001.

Bevorzugt wird in dem erfindungsgemäßen Verfahren die Cyclisierung in Gegenwart einer Brönstedtsäure durchgeführt. Bevorzugt werden dabei Brönstedtsäuren mit einem pKₛ-Wert von kleiner 5, besonders bevorzugt kleiner als 2,5, insbesondere kleiner als 0 eingesetzt. Ganz besonders bevorzugt liegt der pKₛ-Wert der Brönstedtsäure zwischen -1,5 und -11.

Eine Reaktivdestillation ist ein dem Fachmann prinzipiell bekanntes chemisches Verfahren, bei dem eine ein- oder mehrstufige Destillation mit einer chemischen Reaktion, im vorliegenden Fall einer Cyclisierung, verbunden wird. Das Reaktionsprodukt, im vorliegenden Fall ein cyclischer Enolether der Formeln (I) und/oder (II), wird laufend durch Destillation vom Ausgangsprodukt, einem Keton, abgetrennt.

Bevorzugt wird die Reaktivdestillation bzw. die Cyclisierungsreaktion in einem Temperaturbereich zwischen 50 °C und 300 °C, besonders bevorzugt zwischen 80 °C und 200 °C durchgeführt.

In Abhängigkeit von den Siedepunkten der zu trennenden Verbindungen kann der Fachmann üblicherweise unmittelbar oder nach wenigen Versuchen bezüglich der verwendbaren Destillationskolonnen, dem notwendigen Trennleistungsvermögen einer solchen Kolonne sowie den Destillationsparametern wie beispielsweise Druck, Temperatur und Rücklaufverhältnis geeignete Maßnahmen ergreifen, beziehungsweise eine geeignete Auswahl treffen, um das erfindungsgemäße Verfahren in der gewünschten Weise durchführen zu können.

Die Substituenten gemäß der vorliegenden Erfindung sind, soweit nicht weiter eingeschränkt, wie folgt definiert:
Der Begriff "Alkyl", wie vorliegend verwendet, beinhaltet lineare oder ein- bzw. ggf. auch mehrfach verzweigte gesättigte Kohlenwasserstoffe, die auch cyclisch sein können. Bevorzugt ist ein C₁-C₁₀-Alkyl, wie Methyl, Ethyl, n-Propyl, n-Butyl, n-Pentyl, n-Hexyl, n-Heptyl, n-Octyl, n-Nonyl, n-Decyl, Cyclopentyl, Cyclohexyl, Isopropyl, Isobutyl, Isopentyl, Isohexyl, sec-Butyl oder tert-Butyl.

Der Begriff "gesättigter heterocyclischer Rest", wie vorangehend verwendet, bezeichnet beispielsweise mono- oder polycyclische, substituierte oder unsubstituierte Kohlenwasserstoffreste, in denen ein oder mehrere Kohlenstoffatome, CH-Gruppen und / oder CH₂-Gruppen durch Heteroatome, vorzugsweise ausgewählt aus der Gruppe bestehend aus den Elementen O, S, N und P, ersetzt sind. Bevorzugte Beispiele für substituierte oder unsubstituierte gesättigte heterocyclische Reste sind Pyrrolidinyl, Imidazolidinyl, Pyrazolidinyl, Piperidyl, Piperazinyl, Morpholinyl, Tetrahydrofuranyl, Tetrahydropyranyl, Tetrahydrothiophenyl und dergleichen, sowie mit Methyl-, Ethyl-, Propyl-, Isopropyl- und tert-Butylresten substituierte Derivate davon.

Der Begriff "Aryl", wie vorangehend verwendet, bezeichnet beispielsweise aromatische und gegebenenfalls auch kondensierte polyaromatische Kohlenwasserstoffreste, die gegebenenfalls mit linearem oder verzweigtem C₁-C₁₀-Alkyl, C₁-C₁₀-Alkoxy, C₂-C₁₀-Alkenyl oder Halogen, insbesondere Fluor, ein- oder mehrfach substituiert sein können. Bevorzugte Beispiele für substituierte und unsubstituierte Arylreste sind insbesondere Phenyl, Fluorphenyl, 4-Methylphenyl, 4-Ethylphenyl, 4-n-Propylphenyl, 4-Isopropylphenyl, 4-tert-Butylphenyl, 4-Methoxyphenyl, 1-Naphthyl, 9-Anthryl, 9-Phenanthryl, 3,5-Dimethylphenyl, 3,5-Di-tert-butylphenyl oder 4-Trifluormethylphenyl.

Der Begriff "heteroaromatischer Rest", wie vorangehend verwendet, bezeichnet beispielsweise aromatische Kohlenwasserstoffe, in denen ein oder mehrere Kohlenstoffatome durch Stickstoff-, Phosphor-, Sauerstoff- oder Schwefelatome oder Kombinationen davon ersetzt sind. Diese können wie die Arylreste gegebenenfalls mit linearem oder verzweigtem C₁-C₁₀-Alkyl, C₁-C₁₀-Alkoxy, C₂-C₁₀-Alkenyl oder Halogen, insbesondere Fluor, ein- oder mehrfach substituiert sein. Bevorzugte Beispiele sind Furyl, Thienyl, Pyrrolyl, Pyridyl, Pyrazolyl, Imidazolyl, Oxazolyl, Thiazolyl, Pyrimidinyl, Pyrazinyl und dergleichen, sowie mit Methyl-, Ethyl-, Propyl-, Isopropyl- und tert-Butylresten substituierte Derivate davon.

Der Begriff "Arylalkyl", wie vorangehend verwendet, bezeichnet beispielsweise arylhaltige Substituenten, deren Arylrest über eine Alkylkette mit dem entsprechenden Rest des Moleküls verknüpft ist. Beispiele sind Benzyl, substituiertes Benzyl, Phenethyl, substituiertes Phenethyl und dergleichen.

Die Reste R¹ und R² stehen zusammen für eine zweibindige Gruppe -(CH₂)ₓ-, wobei x eine ganze Zahl von 3 bis 12, bevorzugt 4 bis 12, insbesondere 10 bedeutet.

Der Indice m ist gleich null (0), eins (1) oder zwei (2). Im Falle von Verbindungen der Formel (I) ist m bevorzugt eins (1) oder zwei (2) und im Falle von Verbindungen der Formel (II) ist m bevorzugt null (0) oder eins (1). Ganz besonders bevorzugt ist m gleich eins (1) oder zwei (2), insbesondere gleich eins (1).

Die Bildung entweder des cyclischen Enolethers der Formel (I) und/oder der Formel (II) ausgehend von einer bestimmten Ausgangsverbindung der Formel (III) hängt maßgeblich davon ab, an welchem Kohlenstoffatom der Doppelbindung in der Ausgangsverbindung, entweder das den Rest R³ oder das die Reste R⁴ und R⁵ tragende Kohlenstoffatom, sich durch Protonierung der Doppelbindung formal das stabilere Carbokation ausbilden kann. Beispielsweise bilden sich in den Fällen, in denen m gleich 1, R³ gleich Methyl und R⁴ sowie R⁵ gleich Wasserstoff sind, cyclische Enolether der Formel (I) aus, und in den Fällen, in denen m gleich 1, R³ gleich Wasserstoff und R⁴ sowie R⁵ gleich Methyl sind, bilden sich cyclische Enolether der Formel (II) aus.

Bevorzugt sind in dem erfindungsgemäßen Verfahren die Indices in den Formeln (I), (II) und (III) wie folgt definiert.

Der Rest R¹ ist ein C₁-C₁₀- bevorzugt C₁-C₄-Alkylrest, bevorzugt ein linearer C₁-C₁₀- bevorzugt C₁-C₄-Alkylrest.

Der Rest R² ist ein C₁-C₁₀- bevorzugt C₁-C₄-Alkylrest, bevorzugt ein linearer C₁-C₁₀- bevorzugt C₁-C₄-Alkylrest.

Alternativ stehen die Reste R¹ und R² zusammen für eine zweibindige Gruppe -(CH₂)ₓ-, wobei x eine ganze Zahl von 3 bis 12, bevorzugt 4 bis 12, insbesondere 10 bedeutet.

Der Rest R³ ist Wasserstoff oder Methyl.

Der Rest R⁴ ist Wasserstoff oder Methyl.

Der Rest R⁵ ist Wasserstoff oder Methyl.

Der Indice m ist gleich null (0), eins (1) oder zwei (2). Im Falle von Verbindungen der Formel (I) ist m bevorzugt eins (1) oder zwei (2) und im Falle von Verbindungen der Formel (II) ist m bevorzugt null (0) oder eins (1). Ganz besonders bevorzugt ist m gleich eins (1) oder zwei (2), insbesondere gleich eins (1).

Ebenfalls bevorzugt ist ein erfindungsgemäßes Verfahren wie vorangehend beschrieben, wobei die Ausgangsverbindung der Formel (III), die für eine Verbindung der Formel (IIIa) oder eine Verbindung der Formel (IIIb) steht, 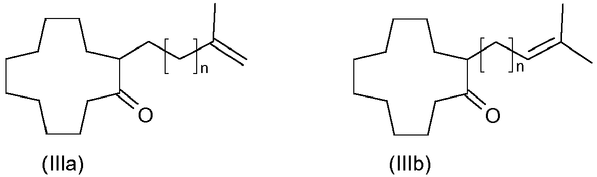 zu dem entsprechenden cyclischen Enolether der Formel (IIa) umgesetzt wird, 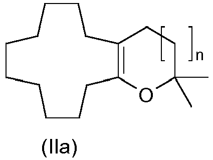 worin in den Formeln (IIa), (IIIa) und (IIIb)
- n: gleich null (0) oder eins (1) ist.

Besonders bevorzugt ist in Verbindungen der Formel (IIIa) n gleich null (0), was zur Bildung des 5-gliedrigen Cycloenolethers führt, und in Verbindungen der Formeln (IIIb) ist n gleich 1, was zur Bildung des 6-gliedrigen Cycloenolethers führt.

Die Erfindung wird durch folgende, die Erfindung jedoch nicht einschränkende Beispiele erläutert.

### Beispiel 1:

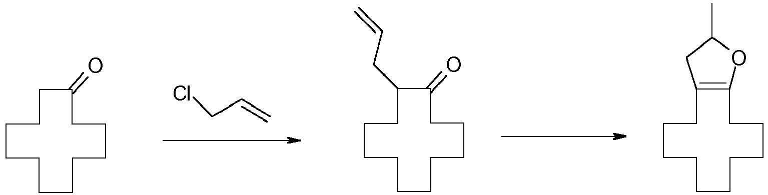

### Allyllierung:

CDon (364,6 g), Toluol (360 ml), Tetrabutylammoniumiodid (7,6 g) und Natronlauge (50%ig, 480 g) wurden in den Reaktor eingefüllt und unter Rühren (400 UpM) auf 90 °C aufgeheizt. Bei 90 °C Innentemperatur wurde mit der Dosierung von Allylchlorid
(306,1 g) begonnen, wobei die Temperatur im Reaktor abfiel und Rückfluss einsetzte. Gesamtdosierdauer: 3 h. Das zweiphasige Reaktionsgemisch wurde dann über Nacht bei 94 °C gerührt. Danach wurde die Reaktionslösung auf RT abgekühlt. Bei 65 °C wurden dabei 500 ml Wasser zugegeben, um den angefallenen Feststoff in der wässrigen Phase zu lösen. Nach der Phasentrennung wurde die organische Phase zweimal mit 500 ml Wasser gewaschen. Danach wurde die organische Phase noch mit 500 g 10%iger Schwefelsäure gewaschen. Die wässrigen Phasen wurden jeweils verworfen.

### Cyclisierung:

213,5 g Allylierungsprodukt werden in einem Destillationskolben mit 5 ml Schwefelsäure versetzt. In der Destillationsapparatur mit 30 cm Füllkörperkolonne (3 mm Metalraschigringe) und Normag-Kolonnenkopf wurde ein Vakuum von 2 mbar angelegt und bei einer Sumpftemperatur von 138-142 °C (Kopftemperatur 108-115 °C) 163,2 g Produkt abdestilliert.

### Beispiel 2:

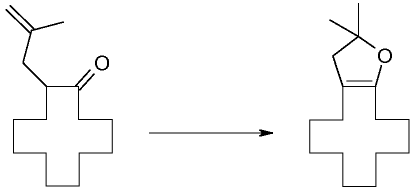

Methallylcyclododecanon wurde ausgehend von CDon und Methallylchlorid, analog wie in Beispiel 1 im Abschnitt Allylierung beschrieben, hergestellt.

### Brönstedt-Säure-Katalyse

In einem 1000 ml Dreihalskolben mit 30 cm Sulzerkolonne und Normag-Kolonnenkopf wurden 1006 g Methallylcyclododecanon vorgelegt und mit 20 g konzentrierter Schwefelsäure versetzt. Es wurde ein Vakuum von 1 mbar angelegt, die Ölbadtemperatur wurde auf 140 °C erhöht. Bei einer Sumpftemperatur von 128-135 °C wurde der Bicyclus langsam aus dem Reaktionsgemisch herausdestilliert (Kopftemperatur 91-96 °C). Insgesamt konnten 866,35 g Produkt aus dem Reaktionsgemisch herausdestilliert werden.

### Lewis-Säure-Katalyse

In einem 500 ml Destillationskolben mit 30 cm Füllkörperkolonne (3 mm Drahtringe) und Normag-Kolonnenkopf wurden 200 g 2-(2-Methallyl-)cyclododecanon vorgelegt und mit 2 g Aluminiumchlorid versetzt. Es wurde ein Vakuum von 2 mbar angelegt, die Ölbadtemperatur wurde langsam auf 175 °C erhöht. Bei einer Sumpftemperatur von 153-156 °C wurde das Produkt aus dem Reaktionsgemisch herausdestilliert (Kopftemperatur 121-123 °C). Insgesamt konnten 140,5 g Produkt aus dem Reaktionsgemisch herausdestilliert werden.

### Beispiel 3:

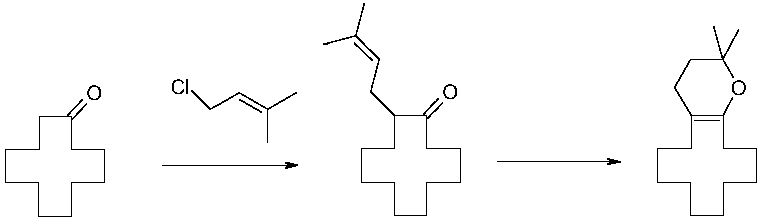

CDon (364,6 g), Toluol (360 ml), Tetrabutylammoniumiodid (7,6 g) und Natronlauge (50%ig, 480 g) wurden in den Reaktor eingefüllt und unter Rühren (400 UpM) auf 90 °C aufgeheizt. Bei 90 °C Innentemperatur wurde mit der Dosierung von 1-Chlor-3-methyl-2-buten (313,7 g) begonnen. Die Temperatur wurde während der gesamten Zugabe bei 90 °C gehalten. Gesamtdosierdauer: 3 h. Das zweiphasige Reaktionsgemisch wurde für 5 h bei 90 °C nachgerührt. Danach wurde die Reaktionslösung auf RT abgekühlt. Bei 65 °C wurden 500 ml Wasser zugegeben, um den angefallenen Feststoff in der wässrigen Phase zu lösen. Nach der Phasentrennung wurde die organische Phase zweimal mit 500 ml Wasser gewaschen. Danach wurde die organische Phase noch mit 500 g 10%iger Schwefelsäure gewaschen. Die wässrigen Phasen wurden jeweils verworfen.

Zu 278 g des Zwischenproduktes wurden 7 g konz. Schwefelsäure gegeben und dann die Lösung in einen 1l Destillationskolben umgefüllt und in einer 70 cm Füllkörperkollonne (3 mm Metalraschigringe) mit Rücklaufteiler bei einer Sumpftemperatur von 185°C einer Kopftemperatur von 130-135 °C einem Druck von 3 mbar und einem Rücklaufverhältnis von 40:1 bis 60:1 destilliert. 191,7 g des Produktes konnten aus der Reaktionsmischung herausdestilliert werden.

### Beispiel 4:

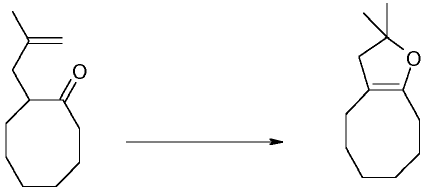

2-Methallylcyclooctanon wurde ausgehend von Cyclooctanon und Methallylchlorid, analog wie in Beispiel 1 im Abschnitt Allylierung beschrieben, hergestellt.

In einem 1 I Destillationskolben mit 30 cm Füllkörperkolonne (3 mm Drahtringe), Normag-Kolonnenkopf und Vakuumregler wurden 302,8 g 2-Methallylcyclooctanon vorgelegt. 3 g konzentrierte Schwefelsäure wurden zugegeben und ein Vakuum von 5 mbar wurde angelegt. Die Ölbadtemperatur wurde langsam auf 130 °C erhöht. Bei einer Sumpftemperatur von 100-105 °C wurde der gebildete Bicyclus langsam aus dem Reaktionsgemisch herausdestilliert. (Kopftemperatur 77 °C). Insgesamt konnten 228,2 g Bicyclus mit einer Reinheit von >96% als Fraktionen erhalten werden.

### Beispiel 5:

3-Methallyl-4-heptanon wurde ausgehend von 4-Heptanon und Methallylchlorid, analog wie in Beispiel 1 im Abschnitt Allylierung beschrieben, hergestellt.

In einem 1 I Destillationskolben mit 30 cm Füllkörperkolonne (3 mm Drahtringe), Normagkolonnenkopf und Vakuumregler wurden 268 g 3-Methallyl-4-heptanon vorgelegt und mit 2,68 g konzentrierter Schwefelsäure versetzt. Ein Vakuum von 20 mbar wurde angelegt und die Ölbadtemperatur auf 110 °C erhöht. Bei einer Sumpftemperatur von 83-88 °C wurde das gebildete Dihydrofuran aus dem Reaktionsgemisch herausdestilliert (Kopftemperatur 67-69 °C). Insgesamt konnten 208,1 g Produkt erhalten werden.

Die Herstellung cyclischer Enolether, insbesondere zu Furanderivaten, findet häufig Anwendung bei der Herstellung pharmazeutischer Wirkstoffe. Die Internationale Anmeldung WO 2008/066299 offenbart die Cyclisierung zu Pyran- und Furannapphthochinonen aus Allylnaphthochinonderivaten , die WO 2008/133441 die Cyclisierung zu Furanphenanthrenchinonen. Bekannt ist die Herstellung von antiproliferativen Naphthochinonen durch säurekatalysierte Cyclisierung von Lapachol oder 5-Hydroxyapachol (Evelyn L. Bonifazi et al.: "Antiproliferative activity of synthetic naphthoquinones related to lapachol. First synthesis of 5-hydroxylapachol", Bioorganic & Medical Chemistry, Bd.18, Nr.7, 1. April 2010, Seiten 2621-2630). Die Synthese dieser Wirkstoffe ist jedoch in der Regel umfangreich und erfordert mehrere Schritte mit einer zur Reinigung anschließenden Destillation.

Mehrere Schritte umfasst auch die Säure katalysierte Cyclisierung von Heptanon zu Pyran (Michele Danet et al.: "Enantioselective Synthesis oft he Originally Proposed Usneoidone Structure: Evidence for a Structural Revision; Eur. J. Org. Chem., Bd. 2004, Nr. 9, 1. Mai 2004, Seiten 1911-1922) oder die Säure katalysierte Cyclisierung zu Furanderivaten, die im Öl der Römischen Kamille vorkommen (Alan F. Thomas et al.:"Homologues of p-Menthane Derivatives in Roman Camomille", Helv. Chim. Acta, Bd. 64, Nr.5, 22. Juli 1081, Seiten 1488-1495).
Die US Patentschrift US 4 056 541 A offenbart die Herstellung von 14, 14-dimethyl-13-oxa-1,12-didehydrobicyclo10.4.0]-hexadecan aus 2-3-methyl-but-2-en-1-yl]-cyclododecanon in Gegenwart von Schwefelsäure. Allen Cyclisierungen schließt sich eine Destillation zur Reinigung des jeweiligen Produktes an.

## Patentansprüche

1. Verfahren zur Herstellung von cyclischen Enolethern der Formeln (I) und/oder (II) 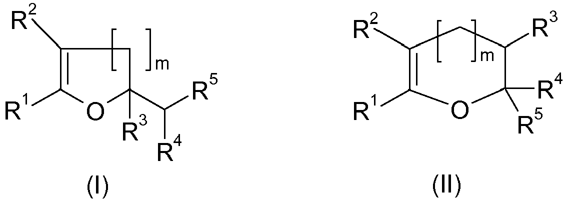 durch Cyclisierung einer Ausgangsverbindung der Formel (III) 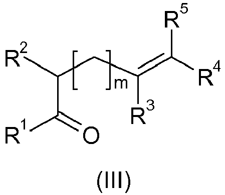 worin
m gleich null (0), eins (1) oder zwei (2) ist,
R¹ ein C₁-C₁₀-Alkylrest ist,
R² ein C₁-C₁₀-Alkylrest ist,
oder die Reste R¹ und R² zusammen für eine zweibindige Gruppe -(CH₂)ₓ- stehen, wobei x eine ganze Zahl von 3 bis 12 bedeutet,
R³ Wasserstoff oder Methyl ist,
R⁴ Wasserstoff oder Methyl ist, und
R⁵ Wasserstoff oder Methyl ist.
in Gegenwart einer Brönstedt- oder Lewis-Säure, wobei die Reaktion als Reaktivdestillation durchgeführt wird, wobei die gebildeten cyclischen Enolether der Formeln (I) und/oder (II) destillativ aus dem Reaktionsgemisch von der Ausgangsverbindung der Formel (III) abgetrennt werden.

2. Verfahren gemäß Anspruch 1, wobei das molare Verhältnis der Brönstedt- oder Lewis-Säure zur Verbindung der Formel (III) nicht größer als 1 ist.

3. Verfahren gemäß Anspruch 1 oder 2, wobei die Cyclisierung in Gegenwart einer Brönstedtsäure mit einem pKₛ-Wert von kleiner 5 durchgeführt wird.

4. Verfahren gemäß einem der Ansprüche 1 bis 3, wobei die Ausgangsverbindung der Formel (III), die für eine Verbindung der Formel (IIIa) oder eine Verbindung der Formel (IIIb) steht, 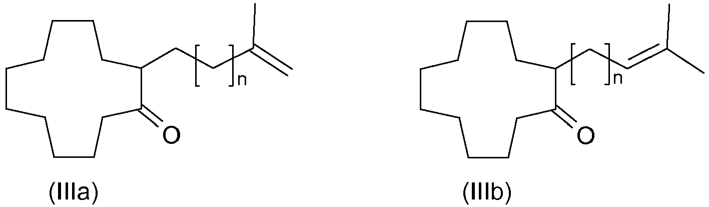 zu dem entsprechenden cyclischen Enolether der Formel (IIa) umgesetzt wird, 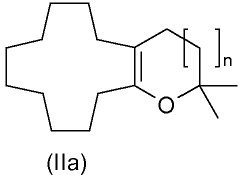 worin in den Formeln (IIa), (IIIa) und (IIIb)
n gleich null (0) oder eins (1) ist.

## Claims

1. A process for the preparation of cyclic enol ethers of the formulae (I) and/or (II) 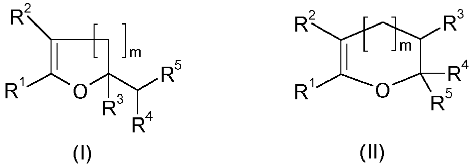 by cyclization of a starting compound of the formula (III) 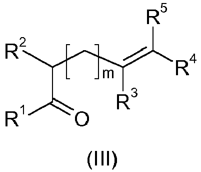 in which
m is zero (0), one (1) or two (2),
R¹ is a C₁-C₁₀-alkyl radical,
R² is a C₁-C₁₀-alkyl radical,
or the radicals R¹ and R² together are a divalent group -(CH₂)ₓ-, where x is an integer from 3 to 12,
R³ is hydrogen or methyl,
R⁴ is hydrogen or methyl, and
R⁵ is hydrogen or methyl,
in the presence of a Brönstedt acid or Lewis acid, where the reaction is carried out as reactive distillation, where the formed cyclic enol ethers of the formulae (I) and/or (II) are separated off from the starting compound of the formula (III) by distillation from the reaction mixture.

2. The process according to claim 1, where the molar ratio of the Brönstedt acid or Lewis acid to the compound of the formula (III) is not greater than 1.

3. The process according to claim 1 or 2, where the cyclization is carried out in the presence of a Brönstedt acid with a pKa value of less than 5.

4. The process according to any one of claims 1 to 3, where the starting compound of the formula (III), which is a compound of the formula (IIIa) or a compound of the formula (IIIb), 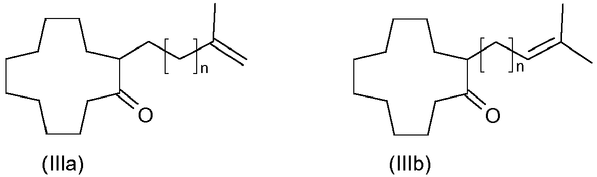 is reacted to give the corresponding cyclic enol ether of the formula (IIa), 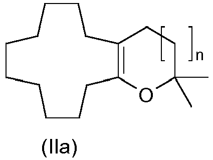 in which, in the formulae (IIa), (IIIa) and (IIIb)
n is zero (0) or one (1).

## Revendications

1. Procédé pour la préparation d'énoléthers cycliques des formules (I) et/ou (II) : 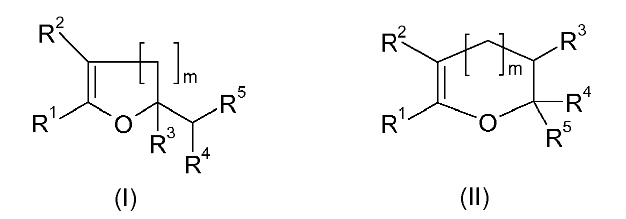 par cyclisation d'un composé de départ de formule (III) 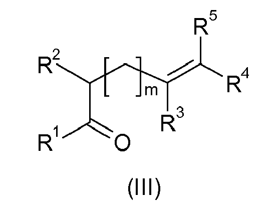 dans laquelle
m vaut zéro (0), un (1) ou deux (2),
R¹ représente un radical C₁-C₁₀-alkyle,
R² représente un radical C₁-C₁₀-alkyle ou les radicaux R¹ et R² représentent ensemble un groupe divalent -(CH₂)ₓ-, x signifiant un nombre entier de 3 à 12,
R³ représente hydrogène ou méthyle,
R⁴ représente hydrogène ou méthyle et
R⁵ représente hydrogène ou méthyle,
en présence d'un acide de Brönstedt ou de Lewis, la réaction étant réalisée sous forme de distillation réactive, les énoléthers cycliques formés des formules (I) et/ou (II) étant séparés par distillation à partir du mélange réactionnel du composé de départ de formule (III).

2. Procédé selon la revendication 1, le rapport molaire de l'acide de Brönstedt ou de Lewis au composé de formule (III) n'étant pas supérieur à 1.

3. Procédé selon la revendication 1 ou 2, la cyclisation étant réalisée en présence d'un acide de Brönstedt présentant une valeur pKₐ inférieure à 5.

4. Procédé selon l'une quelconque des revendications 1 à 3, le composé de départ de formule (III), qui représente un composé de formule (IIIa) ou un composé de formule (IIIb) 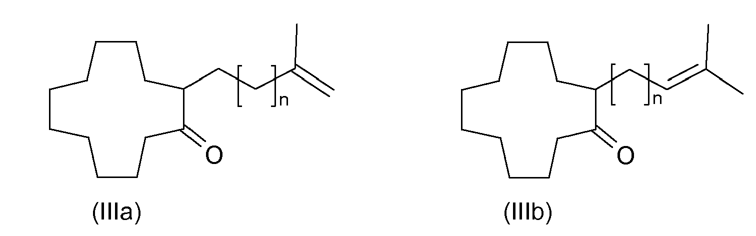 étant transformé en énoléther cyclique correspondant de formule (IIa), 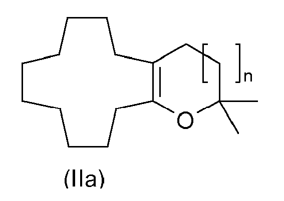 où, dans les formules (IIa), (IIIa) et (IIIb)
n vaut zéro (0) ou un (1).
